# EUROPEAN PATENT SPECIFICATION

(11) **EP 1 948 115 B1**
(45) Date of publication and mention of the grant of the patent: **12.06.2013**
(21) Application number: 06837467.7
(22) Date of filing: 09.11.2006
(51) Int. Cl.: A61K 6/00

(54) **DENTURE ADHESIVE ARTICLES**
GEBISS-KLEBEARTIKEL
ARTICLES ADHESIFS POUR PROTHESE DENTAIRE

(30) Priority: 09.11.2005 US 735243 P; 09.11.2005 US 735088 P; 09.11.2005 US 735136 P; 09.11.2005 US 735135 P; 09.11.2005 US 734874 P; 20.01.2006 US 760528 P; 20.01.2006 US 760660 P; 20.01.2006 US 760516 P; 20.01.2006 US 760526 P; 20.01.2006 US 760527 P; 20.01.2006 US 760711 P; 31.10.2006 US 590145; 31.10.2006 US 590232; 31.10.2006 US 590224; 31.10.2006 US 590233; 31.10.2006 US 590111; 31.10.2006 US 590225; 31.10.2006 US 590191; 31.10.2006 US 590231
(43) Date of publication of application: 30.07.2008
(73) Proprietor: The Procter & Gamble Company, Cincinnati, OH 45202 (US)
(72) Inventor: RAJAIAH, Jayanth, Loveland, Ohio 45140 (US); CAPPEL, Barbara, Ann, Monroe, Ohio 45050 (US); CERDA, Luisa, Navarro, Cincinnati, Ohio 45242 (US); GUO, Judy, Baltimore, Maryland 21224 (US); MEDEIROS, Franco, Silva, Loveland, Ohio 45140 (US)
(74) Representative: Joos, Uta Susanne
(86) International application number: PCT/US2006/044029
(87) International publication number: WO 2007/056606

(56) References cited:
- WO-A-01/15657
- WO-A-01/21093
- WO-A-01/41710
- WO-A-02/30317
- WO-A-96/25910
- WO-A-2005/081935

## Description

### BACKGROUND OF THE INVENTION

Ordinary removable dentures, dental plates and the like, comprise teeth mounted in a suitable plate or base. While dentures are traditionally fitted for the individual user, the fit can change over time which may result in slippage or discomfort. Denture adhesives are used to temporarily adhere the dentures to the surfaces of the oral cavity, in particular the oral mucosa. Denture adhesives are typically applied to either the denture or oral surface at the beginning of the day when the dentures are placed into the oral cavity, and the adhesives tend to bio-erode during the course of the day due to the action of saliva and chewing.

Considerable effort has been made over the years to develop improved denture adhesive products. Both synthetic and natural polymers and gums have been used alone and in combination with various adhesives and other materials in an attempt to improve hold and reduce oozing of the adhesive from under the dental plate, and to avoid messiness and difficulty of removing the residual adhesive from the mouth and dentures after use. For example, alkyl vinyl ether-maleic copolymers and salts thereof are known for providing hold in denture adhesive compositions. Such disclosures include: WO 2002/30317 A, Gasman et al., U.S. Patent 3,003,988, Germann et al., issued October 10, 1961; U.S. Patent 4,980,391, Kumar et al., issued December 25, 1990;. U.S. Patent 5,073,604, Holeva et al., issued December 17, 1991; U.S. Patent 5,525,652, Clarke, issued June 11, 1996; U.S. Patent 5,340,918, Kittrell et al., issued Aug. 23, 1994; U.S. Patent 5,830,933, Synodis et al., issued Nov. 3, 1998. Denture adhesive may be provided as films of any geometric shape (e.g. WO 2001/21093, Govaert).

In addition to adhesion, it is desirable to reduce oozing or to reduce the negative aesthetics of oozing experienced by the consumer. Oozing may occur due to seeping of the denture adhesive from under the dental plate in the oral cavity caused by a variety of factors including a low viscosity denture adhesive, use of too much denture adhesive, improper application of the denture adhesive on the denture plate, etc. When oozing occurs in the oral cavity, the denture adhesive composition is exposed to the oral cavity. Therefore, any negative taste, negative mouth-feel, or any other any other negative aesthetic associated with the denture adhesive composition may be more noticeable and objectionable to the consumer. Sources of such negative perception may include the denture adhesive polymer itself or salts of the denture adhesive polymer, including those crosslinked with zinc salts. Taste considerations are significant since denture adhesive compositions are used in the oral cavity for up to 6-7 hours or longer. Furthermore, consumers may stop using the adhesive or may tend to apply less adhesive the next time if they experience the negative perception of ooze. This may lead to decreased denture hold or decreased denture performance. This decrease in performance can mean less denture stability, denture retention, or an increase in food lodging itself under the denture prosthesis.

In accordance with the present invention, the use of denture adhesive compositions, articles and kits described herein will provide these improved denture adhesive properties including improved hold, fit, ease of handling, ease of application, decreased ooze, and/or improved clean up under the varied conditions of the oral cavity.

### SUMMARY OF THE INVENTION

The invention relates to the use of at least two denture adhesive articles for application to the grooves of a denture, wherein each article is a concave shaped sheet and comprises a homogeneous mixture of:
a) a water insoluble thermoplastic component selected from rubber, elastomers, plastomers, natural wax, synthetic wax, polyvinyl chloride, nylon, fluorocarbon, polyurethane prepolymer, polyethylene, polystyrene, polypropylene, cellulosic resins, acrylic resins, petrolatum, and mixtures thereof;
   and
b) a denture adhesive component.

### BRIEF DESCRIPTION OF THE DRAWINGS

While the specification concludes with claims particularly pointing out ant distinctly claiming the invention, it is believed that the present invention will be better understood from the following description taken in conjunction with the accompanying drawings in which:
Fig. 1 is a top plain view of an embodiment of a concave shaped denture adhesive article having symmetrical dimensions;
Fig. 2 is a top plain view of an embodiment of a concave shaped denture adhesive article having asymmetrical dimensions;
Fig. 3 is a top plain view of an embodiment of a concave shaped denture adhesive article having a kidney shape;
Fig. 4 is a top plain view of an embodiment of a concave shaped denture adhesive article having a kidney shape;
Fig. 5 is a top plain view of an embodiment of a concave shaped denture adhesive article having a kidney shape;
Fig. 6 is a top plain view of an embodiment of a concave shaped denture adhesive article having a kidney shape;
Fig. 7 is a top plain view of an embodiment of a concave shaped denture adhesive article having a kidney shape;
Fig. 8 is a top plain view of an embodiment of a concave shaped denture adhesive article having symmetrical dimensions and having broken fold line markings as the custom fit means.
Fig. 9 is a top plain view of an embodiment of a horseshoe shaped denture adhesive article as comparative example;
Fig. 10 is a top plain view of an upper denture having a horseshoe shaped denture adhesive article placed in the grooves of the lower denture as comparative example;
Fig. 10A is a top plain view of a denture adhesive article shaped to cover substantially all of the surface of an upper denture;
Fig. 10B is a top plain view of a lower denture having a denture adhesive article place across substantially all of the surface of an upper denture;
Fig. 11 is a top plain view of an upper denture having 2 concave shaped article having asymmetrical dimensions placed in the grooves of the lower denture;
Fig. 12 is a top plain view of a lower denture having 2 concave shaped articles having asymmetrical dimensions placed in the grooves of the upper denture;
Fig. 13 is a top plain view of an upper denture having 2 concave shaped articles having symmetrical dimensions placed in the grooves of the lower denture;
Fig. 14 is a top plain view of a lower denture having 2 concave shaped articles having symmetrical dimensions placed in the grooves of the upper denture;
Fig. 15 is a top plain view of an upper denture having three concave shaped articles having symmetrical dimensions placed on the upper denture;
Fig. 16 is a front view of an outer package further comprising a use indicia representing the application of the article onto the denture without pre-wetting the article.

### DETAILED DESCRIPTION OF THE INVENTION

A detailed description of essential and optional components of the present invention is given below.

### DEFINITIONS

The abbreviation "cm", as used herein, means centimeter. The abbreviation "mm" as used herein, means millimeter. The abbreviation "g" as used herein, means gram.

The term "denture" and/or "denture prosthesis" as used herein refers to either the upper or lower denture, or both.

The term "denture adhesive article" and/or "article" as used herein refers to articles designed to fit, conform and adhere to contoured surfaces, such as a denture, as well as the gums or the roof of the mouth. The articles herein are substantially solid prior to use and can be picked up manually in substantially one piece and positioned on the denture.

The term "flexible" or "flexible article" as used herein means that a 0.67 mm thick piece of the article may be wrapped 180 degrees around a solid cylinder of 1 cm diameter without cracking upon visual observation.

The term "safe and effective adhesive amounts" as used herein means an amount sufficient to provide adherence to the oral cavity and/or provide adherence of a denture to the oral cavity, without toxicity to the user or damage to oral tissue.

By "safe and effective amount", as used herein, is meant an amount of an agent high enough to significantly (positively) modify the condition to be treated or positively modify the benefit sought, but low enough to avoid serious side effects (at a reasonable benefit/risk ratio), within the scope of sound medical/dental judgment. The safe and effective amount of an agent may vary with the particular condition being treated, the age and physical condition of the patient being treated, the severity of the condition, the duration of treatment, the nature of concurrent therapy, the specific form of the source employed, and the particular vehicle from which the agent is applied.

The term "AVE/MA" as used herein refers to alkyl vinyl ether-maleic acid or anhydride copolymer. The term "mixed polymer salts" or "mixed salts", as used herein, refers to salts of AVE/MA where at least 2 different cations are mixed on the same polymer with each other or with other salts.

The term "free acid", or "FA" component, as used herein, refers either to the unreacted carboxyl groups (-COOH) of AVE/MA copolymer plus any other monovalent cations of carboxyl groups (e.g., COONa) of the polymer. Monovalent cations include Group IA cations, such as sodium, potassium, hydrogen, etc. In one embodiment, the term "free acid" refers to the unreacted carboxyl groups (-COOH) of AVE/MA plus sodium and potassium cations. In another embodiment, the term "free acid" refers only to the unreacted carboxyl groups (-COOH) of the AVE/MA.

The term "toxicologically-acceptable", as used herein, is used to describe materials that are suitable in their toxicity profile for administration to humans and/or animals.

By "non-aqueous", as used herein, is meant that the article does not contain added water but may contain water that is included in another component as supplied commercially by the manufacturer.

The term "water-insoluble" as used herein refers to a material that, when exposed to an excess of water, does not dissolve, but may disperse to varying degrees. In some embodiments the term "water-insoluble" refers to a material that is less than about 10%, 5%, 2%, or 1% soluble in water.

The term "thermoplastic" as used herein refers to a material that melts, softens, and becomes more flexible, extrudable, deformable, shapable, moldable, flowable, processable, and/or changes rheology when exposed to heat. In one embodiment the material generally solidifies, hardens, and/or substantially returns to its original condition, when subsequently cooled.

The term "bioerodible" as used herein means that the article, when exposed to excess of water or saliva, will erode over time due to physical and/or chemical action. The time necessary to erode the article can be any length of time from instantaneous to five days, in one embodiment the time to erode is from about 1 to about 3 days. The article may erode completely or substantially, however ultimately the article will lose its original form and/or integrity. For example, after application and use for at least about 24 hours in the oral cavity the article will not have sufficient product integrity to easily separate or peel, in its original form, from the denture or oral surface. In one embodiment the article bioerodes such that no portion of the article remains on the denture or mouth after the article has been used in the oral cavity for about 24 hours. In another embodiment some portion or residue from the article remains on the denture or oral surface after removing the denture from the oral cavity; however, this portion or residue from the article can be cleaned by brushing away with a toothbrush, but not easily separated from the denture.

The percentages used herein to describe the cationic salt function of the alkyl vinyl ether-maleic acid or anhydride copolymers are defined as the stoichiometric percent of the total initial carboxyl groups reacted on the polymer.

All other percentages used herein are by weight of the article unless otherwise indicated.

All measurements referred to herein are made at 25°C unless otherwise specified.

All percentages, ratios, and levels of ingredients referred to herein are based on the actual amount of the ingredient, and do not include solvents, fillers, or other materials with which the ingredient may be combined as a commercially available product, unless otherwise indicated.

All publications, patent applications, and issued patents mentioned herein are hereby incorporated in their entirety by reference. Citation of any reference is not an admission regarding any determination as to its availability as prior art to the claimed invention.

To the extent that any meaning or definition of a term in this written document conflicts with any meaning or definition of the term in a document incorporated by reference, the meaning or definition assigned to the term in this written document shall govern.

### DENTURE ADHESIVE ARTICLE

The present invention relates to the use of denture adhesive articles that are designed to fit, conform and adhere to contoured surfaces such as a denture as well as the oral surfaces such as the gums or the roof of the mouth.

In one embodiment the articles herein minimize or avoid the problem of premature sticking during application of the article to the denture by the composition and the shape of the denture adhesive article. That is, with some prior art denture adhesive articles, before the article can be properly positioned over a target surface on the denture, inadvertent contact of the article with the denture may cause premature sticking at one or more locations on the denture. This may inhibit proper positioning of the article. Premature sticking may also cause contamination or degradation of the article prior to final positioning on the denture.

In one embodiment the term "dry tack" as used herein means that present articles exhibit minimal and/or no adhesive or cling properties in the dry state until activated by pressure applied by a user. In one embodiment this characteristic permits the present articles to be stored and dispensed in any desired mode without encountering the difficulties of premature clinging or adhering to themselves, and without the need for separate release sheets, liners, spacers, or the like. At the same time, in one embodiment when pressure activated at the desired location and at the desired time, the articles can, in the dry state, exhibit sufficient adhesive properties to form a bond to most plastic surfaces including a denture surface, this bond being sufficiently strong to survive handling of the denture without bond failure. Therefore, in one embodiment the articles herein, in the dry state, adhere to a target denture surface only when pressed thereagainst, thereby minimizing or avoiding this problem of inadvertent adherence during positioning on the denture surface. In one embodiment then, the articles herein do not have to be moistened or wet prior to application to the denture, thus providing a simple and easy way to apply an article to the denture.

In one embodiment the term "dry tack" as used herein means that present articles exhibit minimal and/or no adhesive or cling properties until activated by pressure applied by the user after the article has been warmed by the hands of the user, potentially during the course of application of the article to the denture surface.

In another embodiment the articles herein are non-tacky to the touch prior to application to the denture.

In another embodiment the term "dry tack" as used herein means articles herein in a dry and un-wetted state, are capable of immediate bonding by surface attachment to a dry plastic, polymethyl methacrylate, and/or other denture prosthesis substrate, upon subjecting the article to pressure. In one embodiment the dry article, develops bonding by surface attachment to a dry denture prosthesis substrate upon the application of finger pressure whereby the article remains bonded under its own weight, and the article herein will not remain bonded to this dry substrate under its own weight without using finger pressure to apply the article to the substrate. In one embodiment the force or pressure may be generated by one or more fingers. This force or finger pressure, in one embodiment, may be applied for 1-10 seconds or longer. In another embodiment the bonding of the article to the substrate is maintained from 10 seconds to 3 minutes or longer, in another embodiment from 30 seconds to 1 minute or longer.

In one embodiment the dry tack of the article is from 0.025, 0.1, 1, 10, 100, 1000 gram force/square centimeter to 10, 100, 1000, 10,000, 50,000, 100,000 gram force/square centimeter and any combination thereof.

In one embodiment the dry tack of an article that can be repositioned is from 0.025 grams/force square centimeter to 0.30 grams/force square centimeter, and in another embodiment from 0.025 gram force/square centimeter to 0.25 gram force/square centimeter.

It is reported that the room temperature modulus of any tacky adhesive is *less* than 3 x 10⁶ dynes/cm² when measured at a frequency of 1 Hz. This finding is a criterion for tack and has been given the name "Dahlquist criterion for tack."(Adhesion and Adhesives Technology, by Alphonsus Pocius, 2nd Edition, 2002 Carl Hanser Verlag, Munich).

In one embodiment of the current invention, the article has a modulus greater than the 'Dahlquist criterion for tack' of 3 × 10⁶ dynes/cm². In another embodiment, the article has a shear storage modulus G' (measured in dynes/cm² at a frequency of about 1Hz at about 25C) greater than 5 × 10⁶; in another embodiment greater than 1 × 10⁷; in another embodiment greater than 5 × 10⁷; and in another embodiment greater than 8 × 10⁷.

In one embodiment the article has a shear storage modulus G' (measured in dynes/cm² at a frequency of about 1Hz at about 25C) from 1 × 10⁶, 3 × 10⁶, 5 × 10⁶, 1 × 10⁷, 5 × 10⁷, and 8 × 10⁷ to 5 × 10⁸, 5 × 10⁷, 1 × 10⁸, 5 × 10⁹, 1 × 10⁹, and 1 × 10¹⁰ and/or any combination thereof.

In one embodiment the article has a flexural stiffness of less than 10 grams/cm, in another embodiment less than 5 grams/cm, in another embodiment less than 3 grams/cm, in another embodiment less than 2 grams/cm and in yet another embodiment from 0.1, 0.5, 1, to 2, 3, 5, 10 grams/cm, in any combination, flexural stiffness as measured on a Handle-O-Meter, model #211-300, available from Thwing-Albert Instrument Company of Philadelphia, PA as per test method ASTM D2923-95.

In one embodiment the articles herein have a normalized dislodgement force of from 1100 to 12,000 grams per sq.cm, in another embodiment from 1300 to 10,000 grams per sq.cm, in another embodiment from 1200 to 5000 grams per sq.cm, in another embodiment from 1400 to 5000 grams per sq.cm, in another embodiment from 1300 to 2500 grams per sq.cm, in another embodiment from 1750 to 2500 grams per sq.com. In another embodiment the normalized dislodgement force is from 1100, 1200, 1300, 1400, 1500, 1750 grams per sq.cm. to 12,000, 10,000, 7500, 5000, 2500, 2250 grams per sq.cm, and/or any combination thereof. In one embodiment the dislodgement force ratio is from 1.1, 1.2, 1.3, 1.4, 1.5, 1.6, 1.7, 1.8, 1.9, 2.0 to 1.3, 1.4, 1.5, 1.6, 1.7, 1.8, 1.9, 2.0, 3, 4, 5, 6, 8, 10, and/or any combination thereof. In one embodiment the dislodgement force ratio is from 1.1 to 10, from 1.1 to 8, from 1.3 to 4, and/or from 1.3 to 2.5.

In one embodiment the articles herein are substantially solid prior to use and can be picked up manually, in substantially one piece, and positioned on the denture. Additionally, in one embodiment the articles are capable of being picked up manually, and positioned on the denture, resulting in little or no residue on the fingers. In another embodiment the articles comprise a single layer. In yet another embodiment the articles are laminates and/or composites. In one embodiment the articles are pre-shaped and/or preformed. In another embodiment the articles herein may be dispensed by the consumer via a unit dose package, multi-dose package, pump, sachet, syringe, or tube, and shaped by the consumer; and in yet another embodiment the articles may be shaped by the consumer without leaving a substantial residue on their hands.

In one embodiment, the denture adhesive article is sufficiently flow-able to allow it to be applied from a tube and subsequently picked up and positioned on the denture. In one embodiment, the denture adhesive article is sufficiently flow-able to allow it to be applied from a tube and subsequently picked up, and positioned on the denture, with little/no residue on the fingers. In another embodiment the denture adhesive article comprises a solvent which results in an article that is sufficiently flow-able to be applied from a tube. The solvent may be subsequently dissipated, by evaporation, bio-absorption, dispersion, dissolution, etc. In another embodiment the above mentioned optional solvent is also miscible with the water insoluble component. In one embodiment, the denture adhesive article is sufficiently flow-able to allow it to be applied from a tube and only minimally ooze out from under the denture. In one embodiment the denture adhesive article has a "normalized ooze amount" from 0%, 0.00001%, 0.001%, 5%, 10%, 15%, 20%, 25%, 30% to 15%, 20%, 25%, 30%, 40%, 50% and/or any combination thereof. In one embodiment the denture adhesive article has a "ooze ratio" from 0, 0.00001, 0.001, 0.01, 0.1, 0.2, 0.3, 0.4, 0.5, 0.6 to 0.3, 0.4, 0.5, 0.6, 0.7, and/or any combination thereof

Regardless of the form of dispensing the article, including but not limited to pre-dosed ready to use articles and/or articles which are dispensed such as from a tube, the articles are substantially solid prior to use and can be picked up manually. Denture adhesive articles that can be dispensed from a tube can be identified as articles by the following method:
Please read all steps before starting test.
   1. Fill product into a tube with a 0.16" diameter nozzle.
   2. Extrude a 1" long strip of product onto a denture tile (1.5"x1.5" square tile made from denture-plastic) taking care to hold the nozzle about 1/8" above the denture-tile. Do not touch the nozzle to denture-tile while extruding the product.
   3. After about 1" of product has been extruded, hold nozzle about 1/8" above the denture-tile and use a spatula to cut the strip against the nozzle. Do not touch or smear the nozzle against the denture-tile while cutting strip.
   4. Use thumb and forefinger to hold the middle of the strip and pick it up vertically off the denture-tile. Do not use a wiping motion of the fingers against the denture-tile.
   5. The composition is an article if it can be picked up in substantially one piece.

Some denture adhesive articles are pre-dosed and/or ready to use. A user may be able to identify these items visually as a denture adhesive article, as they are often in the form of a strip contained within a package. However, if not evident that these denture adhesive products are articles, these denture adhesive articles can be identified as articles by the following method:
1. Shape composition into a sheet 0.67 mm thick × 8 mm wide × 44 mm long.
2. Place sheet on a denture-tile.
3. Use fingers to pick up sheet.
4. The composition is an article if it can be picked up in substantially one piece.

Substantially in one piece means, as used herein, that from 75, 80, 85, 90% to 100, 90, 85, 80, 75, 70% and/or any combination thereof of the denture adhesive composition remains in one piece when manually picked up from the denture surface.

In addition to the aforementioned test methods denture adhesive articles can also be identified as articles by the amount of ooze, as determined by the ooze method (as defined herein). In addition to being able to be manually picked up and moved in substantially one piece, a denture adhesive article has a normalized ooze amount of from 0, 3, 5, 10, 15, 20, 25% of the total composition to 30,25,20,15,10, 5, 3 % of the total composition and/or any combination thereof and/or the ooze ratio is from 0, 00001, 0.001,0.01, 0.1,0.2,0.25, 0.3, to 0.1, 0.2, 0.25, 0.3, 0.4, 0.5 and/or any combination thereof.

In one embodiment the article herein comprises ingredients of natural origin.

In one embodiment the article herein comprises a homogeneous mixture of the denture adhesive component and the water insoluble thermo-plastic component.

### Shape of Denture Adhesive Articles

Another means of preventing premature sticking is shaping the article suchathat proper application to the denture or oral surface is self evident. For example, the article can be in the shape of the area into/on to which it is to be placed. The adhesive activity of the denture adhesive article is maximized by providing maximum contact between the oral surface and the denture with the placement of the denture adhesive article. Methods of improving the contact between the article and the denture and/or the oral surface include, but are not limited to, shaping the denture article such that it curves along the shape of the denture and/or the oral surface, and/or shaping the article such that it fits into the grooves of the denture. A shaped article can also result in "self-evident application," which results when an article is shaped to fit a particular area of the denture. A user can look at the article and at the denture and can see where the article will best fit. Improved adhesion can be achieved by shaping the article such that it will best fit into the space that, when an article is applied there, good adhesion results. If the user can see which space looks most like the shape of the article without having to read or follow instructions, the user is most likely to place the article into this space.

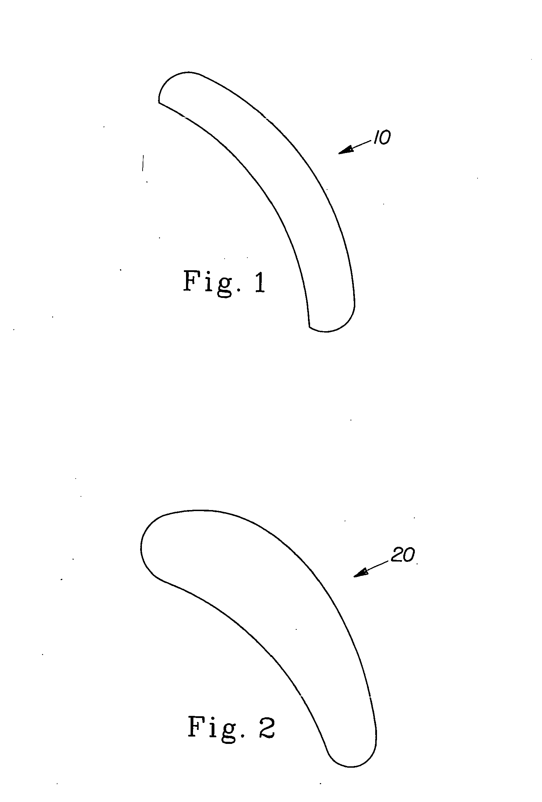

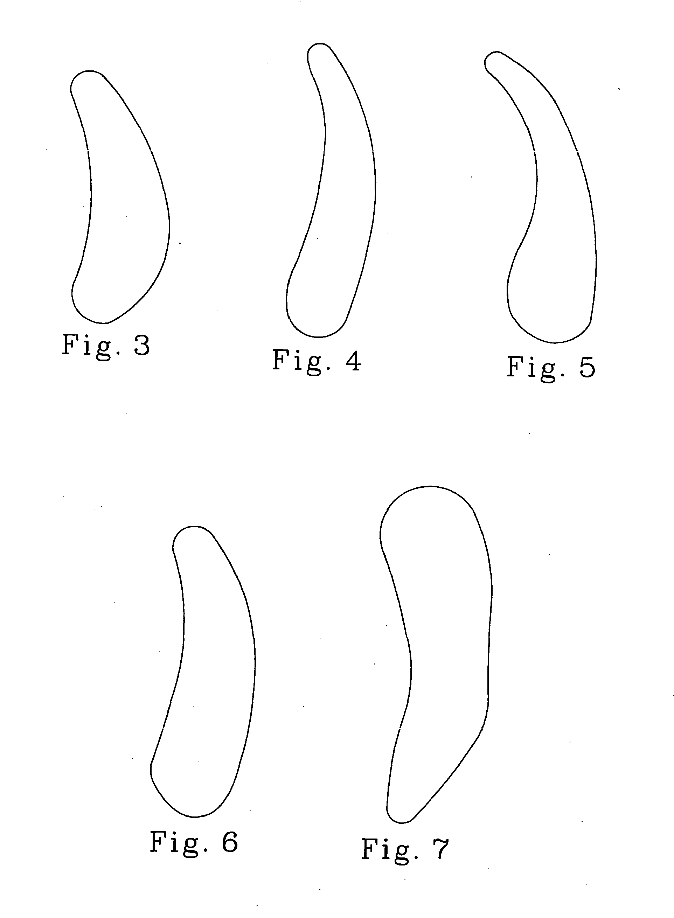

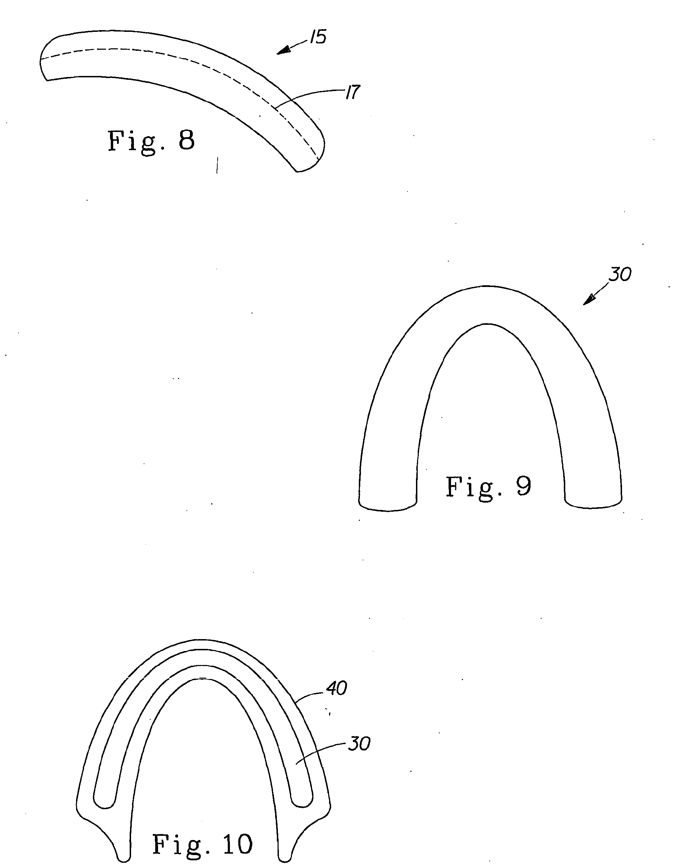

The use according to the present invention includes concaved shaped, denture adhesive articles 10, 20 comprising a safe and effective adhesive amount of a denture adhesive component shown in Figs. 1 and 2. In another embodiment, the concave shaped articles are designed to fit, conform and adhere to contoured surfaces such as a denture as well as the gums or the roof of the mouth. In one embodiment the concaved shaped articles are kidney shaped (as shown in Figs. 2-7). In another embodiment the concave shaped articles herein are hollowed or rounded inward like the inside of a bowl (as shown in Fig. 8). The shape of the article can facilitate ease of alignment of the article in the groove of the denture to result in an improved fit and therefore improved adhesion. Additionally, as shown in Fig. 8 the article can comprise fold lines 17 to facilitate customizing the article to fit the particular denture size and shape of the user.

In one embodiment the articles are from 10, 15, 20, 25, 30, 35, 40 to 25, 35, 40, 45, 50, 55, 60, 100, 110; 120, 140 mm long and/or any combination thereof. In one embodiment the articles are from 35 to 45 mm long, in another embodiment the articles are from 40 to 45 mm long. In comparative embodiments shown in Figs. 9-10, the article 30 is in the shape of the groove that extends the entire length of the denture 40; and can be from 100 to 150 mm long; this shape can be considered a horse-shoe shaped denture adhesive article 30.

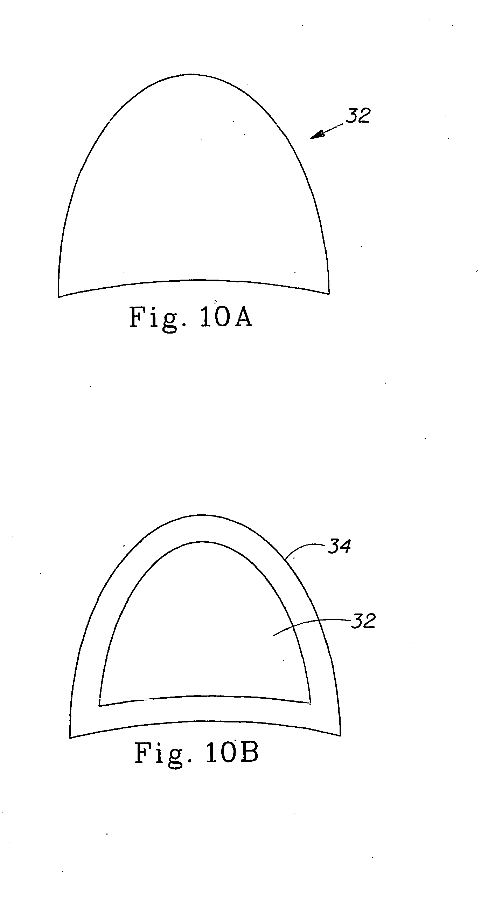

In yet another comparative embodiment, shown in Fig. 10A a denture adhesive article can be shaped to cover substantially all of the surface of an upper denture. This denture adhesive article 32 can be placed across substantially all of the surface of an upper denture 34 (as shown in Fig. 10 B).

The article can be either symmetrical or asymmetrical. In one embodiment the article is asymmetrical, for example kidney shaped (shown in Figs. 2-7), and one end is from 10,12, 15, to 12, 15, 20 mm wide and the article tapers down the length to be from 3, 5, 7, 8, 9 to 5, 8, 10 mm wide at the opposing end. As shown in Figs. 2-7 the kidney shape can be of varying width and the difference between the widest point in width to the narrowest point in width can vary depending on this variation. In another embodiment the article is symmetrical (as shown in Figs. 1 and 8) and is from 4, 5, 6, 8, 10 to 6, 8, 10, 12, 15 mm wide down the entire length of the article.

In one embodiment the thickness of the articles is generally between 0.1 mm to 2.5 mm, in another embodiment is from 0.4 mm to 1.5 mm thick in another embodiment is from 0.5 mm to 1mm thick. The article may be thicker or thinner depending on the degree of cushioning desired by the user or wearer. Multiple denture adhesive articles can be stacked on top of each other to adjust the thickness, degree of cushioning, and/or adhesiveness.

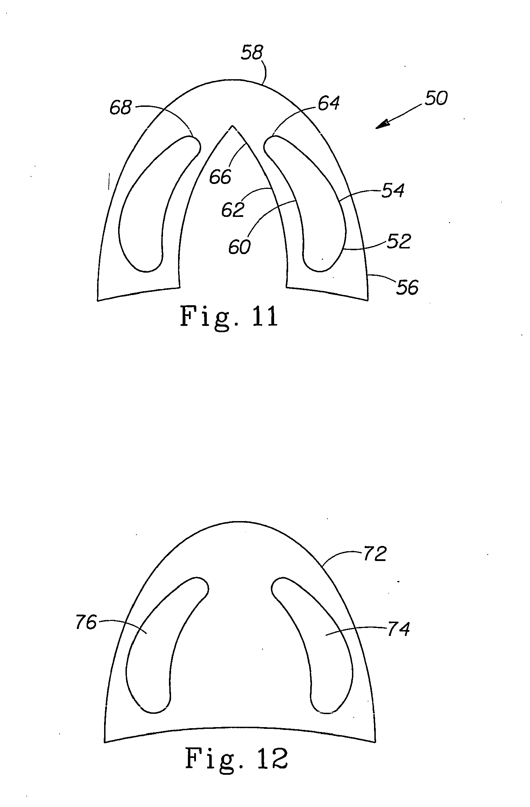

In one embodiment the widest portion 52 of a kidney shaped denture adhesive article 54 is placed into the widest portion of the groove 56 of the denture 58 (as shown in Figs. 11-12). The curved portion 60 of the kidney shaped denture adhesive article 54 follows along the curved portion 62 of the denture 58, and the kidney shaped denture adhesive article 54 continues to taper down in width until the end 64 of the denture adhesive article 54 which is the narrowest in width fits into the narrowest portion 66 of the denture 58. The kidney shaped denture adhesive articles can be placed on an upper denture plate or a lower denture plate (Figs. 11 -12). Fig. 11 shows a lower denture plate 58 comprising two denture adhesive articles 54 and 68. Whereas, Fig. 12 shows an upper denture plate 72 comprising two kidney shaped denture adhesive articles 74 and 76.

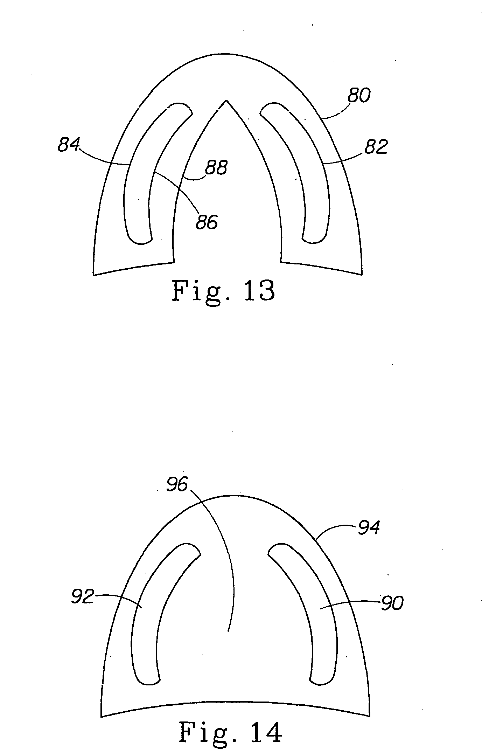

In another embodiment the symmetrical concave shaped denture adhesive articles 82 and 84 can be placed onto a lower denture plate 80 (as shown in Figs. 13-14). The curved portion 86 of the denture adhesive article 84 can follow along the curved portion 88 of the denture 80. The same arrangement can be used for the upper denture plate as shown in Fig. 14. Two denture adhesive articles 90 and 92 are placed along the edge of the denture plate 94.

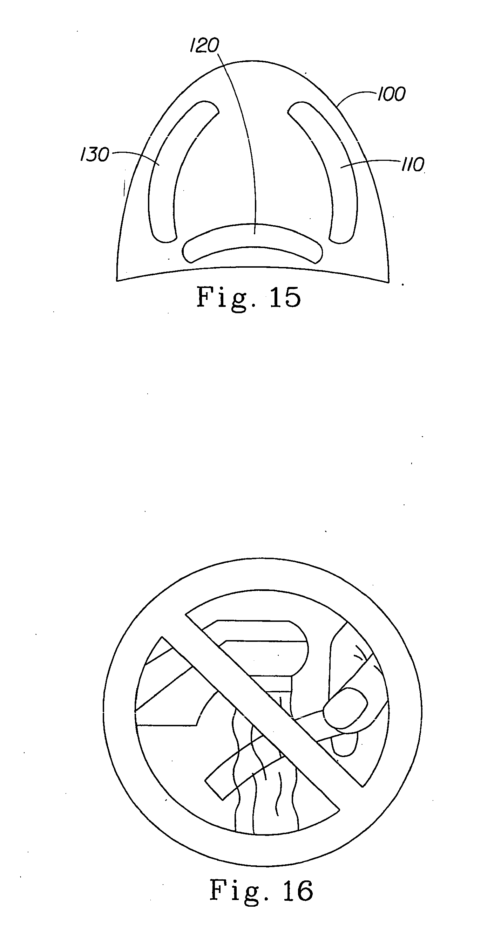

In one embodiment no denture adhesive article is placed across the center portion 96 of the upper denture 94. This can allow for a better fit if the upper denture is fitted closely to the upper palate. In yet another embodiment (as shown in Fig. 15), if desired a denture adhesive article 120 can be placed in the center portion of the upper denture 100, as well placing to denture adhesive articles 110, and 130 along the two sides of the denture plate 100.

In one embodiment the articles herein may optionally be multiphase or have visually distinct phases. In another embodiment the articles herein may optionally have a release liner.

In one embodiment (as shown in Fig. 8) the articles of the present invention further comprise a custom fit means to allow the consumer to trim, cut, fold, tear, reshape, or otherwise custom fit the article to their denture/denture groove size to obtain the desired shape and fit prior to application of the article to the denture. In one embodiment, the custom fit means may be selected from the group consisting of perforations, broken lines, solid lines, shading, markings, creases for folding, and combinations thereof. In yet another embodiment the article 15 comprises a broken fold line 17 along which the user can fold or tear the article to custom fit the article to the denture.

In one embodiment, the present articles are generally applied to the denture prosthesis and thereafter the denture is secured to the oral cavity. In one embodiment the dentures are dried prior to application of the article. In one embodiment it is not necessary to wet or moisten the article prior to applying it to the denture or prior to insertion into the oral cavity for use as a denture adhesive. In one embodiment, the denture wearer generally wears the article from 1 hour to 3 days, in another embodiment from6 hours to 24 hours. In one embodiment, after usage the denture is removed from the oral cavity, and any remaining article may be removed from the prosthesis, for example by gentle scrubbing with water and a brush.

### DENTURE ARTICLE COMPOSITION

### DENTURE ADHESIVE COMPONENT

The present invention comprises a safe and effective adhesive amount of a denture adhesive component, generally at a level of from10% to 90%, in another embodiment from 15% to 70%, in another embodiment from 20% to 70%, in yet and in another embodiment from 25% to 65%, and in yet another embodiment from 30% to 65%, by weight of the article. In one embodiment the articles of the present invention comprise from at least 20 percent by weight, and in another embodiment at least 30 percent by weight of the article, of a denture adhesive component.

In one embodiment the denture adhesive components herein are mucoadhesive, hydrophilic, water soluble, have the property of swelling upon exposure to moisture, and/or to form a mucilaginous mass when combined with moisture. In one embodiment the denture adhesive components are selected from the group consisting of natural gums, synthetic polymeric gums, AVE/MA, salts of AVE/MA, AVE/MA/IB, salts of AVE/MA/IB, copolymers of maleic acid or anhydride and ethylene and salts thereof, copolymer of maleic acid or anhydride and styrene and salts thereof, copolymer of maleic acid or anhydride and isobutylene and salts thereof, polyacrylic acid and polyacrylates thereof, polyitaconic acid and salts thereof, mucoadhesive polymers, water-soluble hydrophilic colloids, saccharide derivatives, cellulose derivatives, and mixtures thereof. Examples of such materials include karaya gum, guar gum, gelatin, algin, sodium alginate, tragacanth, chitosan, acrylamide polymers, carbopol, polyvinyl alcohol, polyamines, polyquarternary compounds, polyvinylpyrrolidone, cationic polyacrylamide polymers, AVE/MA, AVE/MA/IB, mixed salts of AVE/MA, mixed salts of AVE/MA/IB, polymeric acids, polymeric salts, polyhydroxy compounds, and mixtures thereof.

In one embodiment the denture adhesive components are selected from the group consisting of salts of AVE/MA, mixed salts of AVE/MA, cellulose derivatives (such as methylcellulose, carboxymethylcellulose, hydroxyethylcellulose, hydroxypropylcellulose, hydroxy-propylmethylcellulose, and mixtures thereof), polyethylene glycol, karaya gum, sodium alginate, chitosan, corn starch, and mixtures thereof. In yet another embodiment, the adhesive component is selected from the group consisting of mixed salts of AVE/MA, cellulose derivatives, and mixtures thereof.

In one embodiment the denture adhesive component is not thermoplastic and/or comprises only low levels of water soluble thermoplastic polymers, from 0.01 to 5% of water soluble thermoplastic polymer such as polyethylene oxide, hydroxypropyl cellulose, hydroxyproplymethylcellulose; polyethylene glycol; in another embodiment from about 0.01 to about 1% of water soluble thermoplastic polymer, or is essentially free of water soluble thermoplastic polymers.

### Alkyl Vinyl Ether-Maleic Copolymer

In one embodiment of the invention the denture adhesive component is AVE/MA or salts of AVE/MA. The alkyl vinyl ether-maleic acid co-polymer comprises or consists essentially of the repeated structural unit: 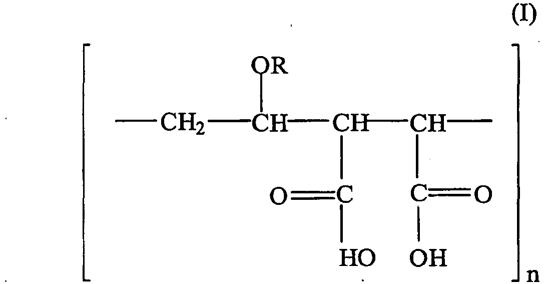 wherein R represents an alkyl radical, in one embodiment a C₁ to C₅ alkyl radical, n is an integer greater than one representing the number of repeated occurrences of the structural unit in a molecule of the polymer.

In one embodiment, the adhesive component is AVE/MA and salts thereof, preferably mixed salts of AVE/MA, wherein the copolymer contains a cationic salt function comprising a cation selected from the group consisting of Group IA and Group 2A cations of the periodic table, yttrium, titanium, zirconium, vanadium, chromium, manganese, iron, nickel, copper, zinc, boron, aluminum, and mixtures thereof. In another embodiment, the adhesive component is a mixed salt of AVE/MA containing a cationic salt function comprising a cation selected from the group consisting of strontium, zinc, iron, boron, aluminum, vanadium, chromium, manganese, nickel, copper, yttrium, titanium, magnesium, calcium, sodium, and mixtures thereof, and in yet another embodiment the cation is selected from the group consisting of strontium, zinc, iron, magnesium, calcium, sodium, and mixtures thereof.

AVE/MA contains, in one embodiment, a cationic salt function comprising from about 5% to about 50%, in another embodiment, from 10% to 40%, in yet another embodiment, from 10% to 35% (of the total initial carboxyl groups reacted) zinc cations. These zinc cations can be mixed with other cations selected from the group consisting of: from 5% to 65%, preferably from 10% to 60%, strontium cations, from 0.001% to 2.5%, preferably from 0.01% to 2% of iron, boron, aluminum, vanadium, chromium, manganese, nickel, copper, yttrium, and/or titanium cations, from 5% to 65%, preferably from 15% to 50% of calcium and/or magnesium cations and/or sodium cations.

AVE/MA and salts thereof, are also described in U.S. Patent Nos. 5,073,604 to Holeva et al., issued 12/17/91; 5,525,652, issued Jun. 11, 1996, Clarke et al.; 6,025,411, issued Feb. 15, 2000, Wong et. al.; 4,758,630, issued July 19, 1988, Shah et al.; 5,304, 616, issued April 19, 1994, Rajaiah et al.; 5,424,058, issued June 13, 1995, Rajaiah; 5,424,058, issued 6/13/95, Rajaiah et al.; 4,758,630, issued July 19, 1988, Shah et al.; 5,830,933, issued Nov. 3, 1998, Synodis et al.; 2,047,398, issued July 14, 1936, Voss et al.; 3,003,988, issued Oct. 10, 1961, Germann et al.; 5,880,172, Rajaiah et al., issued March 9, 1999; 5,900,470, Prosise et al., issued 5/4/99; 5,037,924, Tazi et al., issued 8/6/91; 5,082,913, Tazi et al, issued 1/21/92; all of which are incorporated herein by reference in their entirety. Salts of AVE/MA are also described in P&G copending applications U.S. Patent Nos. 6,355,706 to Rajaiah, et al., issued March 12, 2002; 6,617,374 to Rajaiah, et al., issued September 9, 2003.

In one embodiment the free acid level of the salts of the AVE/MA or AVE/MA/IB is at least 36%, in another embodiment is from 36% to 60%, and even in another embodiment is from 40% to 55%, of the total initial carboxyl groups of the copolymer.

In one embodiment the specific viscosity of the starting copolymer acid or copolymer anhydride is from 1.2 to 14, when preferably measured in a 1% weight/volume solution in MEK (methyl ethyl ketone) at 25°C. Other methods and solvents can be used to measure the specific viscosity such as a 1% weightivolume solution in DMF (dimethyl formamide) at 25°C and a 1% weight/volume solution in 2-butanone at 25°C.

Suitable AVE/MA copolymers may be prepared by well-known methods of the prior art; see, for example, US 2,782,182, and US 2,047,398.

Methods of making mixed salts of AVE/MA polymers are further disclosed in U.S. Patent Nos. 5,073,604, Holeva et al., issued Dec. 17, 1991 and 5,872,161, Liang et al., issued Feb. 16, 1999.

### WATER INSOLUBLE COMPONENT

The present article comprises a safe and effective amount of a water insoluble component. In one embodiment this component is at a level from 2, 5, 10, 20, 25, 30, 35% to 45, 50, 60, 70, 90%, and/or any combination thereof to create ranges, by weight of the article, in another embodiment the water insoluble component level is from 20% to 70%, from 25% to 60%, or from 35% to 60% by weight of the article. In yet another embodiment the water insoluble component is both water insoluble and substantially non-swellable in water.

In one embodiment the water insoluble component is a water insoluble thermoplastic component that is selected from the group consisting of rubber, elastomers, plastomers, natural wax, synthetic wax, polyvinyl chloride, nylon, fluorocarbon, polyurethane prepolymer, polyethylene, polystyrene, polypropylene, cellulosic resins, acrylic resins, petrolatum, and mixtures thereof. In another embodiment the water insoluble thermoplastic component is selected from the group consisting of natural wax, synthetic wax, petrolatum, polyethylene, and mixtures thereof. In yet another embodiment the water insoluble thermoplastic component is selected from the group consisting of polyethylene, petrolatum, paraffin wax, microcrystalline wax, polypropylene, polystyrene, and mixtures thereof; in another embodiment is selected from the group consisting of polyethylene, microcrystalline wax, and mixtures thereof. In one embodiment the water insoluble thermoplastic component comprises elastomers such as Ethylene-Ethylene-Propylene rubber, Ethylene-Propylene rubber, Styrene-Ethylene-Ethylene-Ethylene-Propylene-Styrene rubber, and combinations thereof; and these may optionally be further combined with waxes.

In one embodiment the article herein has a water insoluble thermoplastic component having a penetration of 25 to 350; in another embodiment from 27 to about 250 in another embodiment from 30 to 150, and in another embodiment from 40 to 150, and in another embodiment from 50 to 80, and in yet another embodiment from 60 to 80. The penetration values are from 25 to 250 when measured using the ASTM D1321 method and the penetration values are from 25 to 350 when measured using the ASTM D937 method, both are existing methods known in the art. Although ASTM D937 and ASTM D1321 generally are used to measure the penetration of petrolatum or petroleum waxes, respectively, these methods may be used to measure the penetration of waxes derived from petroleum and other types of water insoluble thermoplastic components with appropriate modifications, for example, these other components may need to be melted at higher temperature which will be apparent to one of skill in the art.

In obtaining the above penetration values, water insoluble thermoplastic component that are outside of these penetration values may be mixed with another ingredient that modifies the penetration of the thermoplastic component. Therefore the water insoluble thermoplastic component may be a single ingredient or may be a mixture of ingredients. For example, Multiwax W 180 manufactured by Witco (Crompton, Sonneborn), having a penetration value of about 15 to about 20 may be mixed with petrolatum (at a 1:1 ratio) to raise the penetration value to above 25.

In one embodiment the water insoluble thermoplastic component is a natural or synthetic wax. These waxes include natural waxes such as animal, vegetable, mineral wax. Animal waxes include beeswax, lanolin, shellac wax, Chinese wax, etc. Vegetable waxes include carnauba, candelilla, bayberry, sugar cane, etc., and mineral waxes include fossil or earth waxes (ozocerite, ceresin, montan), and petroleum waxes such as paraffin, microcrystalline, etc. In one embodiment the waxes herein are natural waxes selected from the group consisting of beeswax, candelilla, candela, carnauba, paraffin, microcrystalline wax, Fischer-Tropsch waxes, and mixtures thereof.

In another embodiment the wax is microcrystalline wax manufactured by Crompton, Sonneborn (Witco) and referred to and sold under the trademark MutiwaxW-835. This wax has a melt point ranging from 73.9 ° C to 79.4. ° C (ASTM D127), has a penetration at 25 ° of from 60 to 80 (ASTM D1321), has a kinematic viscosity at 98.9 ° C, of from 75 to 90 (ASTM D2161), has a flash point, COC, of about 246 ° C Min. (ASTM D92), and has a congealing point, of about 77 ° C (ASTM D938).

In one embodiment the paraffin waxes useful herein generally can have a melting point range of from 65° C to 80° C, in another embodiment 70° C to 75° C; the microcrystalline wax useful herein can have a melting point of from 65° C to 90° C, in another embodiment can have a melting point of from 80° C to 90° C; the beeswax useful herein can have a melting point of from 62° to 65° C with a flash point of 242° C; the candelilla wax useful herein can have a melting point of from about 68° to about 72° C; the carnauba wax useful herein can have a melting point of from 83° to 86° C; the Fischer-Tropsch wax useful herein can have a melting point of 95° to 120° C. Synthetic grades of beeswax, candelilla, and carnauba waxes are also available with similar properties as the natural grades.

In another embodiment the water insoluble thermoplastic component is polyethylene such as A-C 1702 and A-C 6702 made by Honeywell, with a penetration value of 98.5 and 90.0, respectively, under ASTM D 1321.

In one embodiment the article contains polyethylene oxide, and the water insoluble component is thermoplastic. In another embodiment the article contains polyethylene oxide and the article may not include a fibrous paper web or paper laminate.

In one embodiment the article herein is substantially free of honey mixed with alcohol. In another embodiment the article is substantially free of polyvinyl acetate resin in ethyl alcohol.

### MISCELLANEOUS OPTIONAL INGREDIENTS

### Plasticizing Agent

The articles of the present invention may also optionally comprise a safe and effective amount of one or more toxicologically-acceptable plasticizers. In one embodiment the level of the plasticizing agent ranges from 0.0% to 40%, in one embodiment from 0.01 % to 40%, in another embodiment from 1% to 10%, in another embodiment from 2% to 5%, by weight of the article. In yet another embodiment the denture adhesive article does not comprise a plasticizer.

Suitable plasticizing agents of the present invention include, but are not limited to, polyols (such as sorbitol); glycerin; propylene glycol; acetylated monoglyceride; hydrogenated starch hydrolysates; corn syrups; and derivatives thereof; xylitol, glycerol monoesters with fatty acids; triacetin; diacetin; and monoacetin; dimethyl phthalate; diethyl phthalate; dioctyl phthalate; diethylene glycol; triethylene glycol; tricresyl phosphate; dimethyl sebacate; ethyl glycolate; ethylphthalyl ethyl glycolate; o- and p-toluene ethyl sulfonamide; phthalic acid derivative, glycerol triacetate, citric acid derivative, phosphoric acid derivative, glycol, glycol derivative, paraffin wax, a pentaerythritol ester of a fatty acid, stearic acid derivative, glycerol monostearate, polyethylene glycol, butyl phthalyl butyl glycolate, butyl phthalyl butyl glycolate, dimethyl phthalate, dibutyl phthalate, triacetin, triethyl citrate, acetyl triethyl citrate, acetyl tributyl citrate, triphenyl phosphate, diethylene glycol, caprylic triglyceride, capric triglyceride, propylene glycol dicaprylate/caprate and/or combinations thereof.

In another embodiment the plasticizer is water insoluble.

In one embodiment, the denture adhesive article, when extruded thermoplastically, does not cure and set as a result of the action of the plasticizer component. In another embodiment the plasticizer component does not solidify the water insoluble component or the denture adhesive article. In another embodiment the water insoluble thermoplastic component does not cure and set.

Alternatively, in one embodiment the denture adhesive article may be substantially free of plasticizers. In one embodiment the denture adhesive article can be substantially free of polyethylmethacrylate, triacetin, phthalic acid derivative, glycerol triacetate, citric acid derivative, phosphoric acid derivative, glycol, glycol derivative, paraffin wax, a pentaerythritol ester of a fatty acid, stearic acid derivative, glycerol monostearate, polyethylene glycol, butyl phthalyl butyl glycolate, butyl phthalyl butyl glycolate, dimethyl phthalate, dibutyl phthalate, triacetin, triethyl citrate, acetyl triethyl citrate, acetyl tributyl citrate, triphenyl phosphate, diethylene glycol, caprylic triglyceride, capric triglyceride, propylene glycol dicaprylate/caprate and/or combinations thereof.

### Gellant Agents

The articles of the present invention may also optionally comprise a safe and effective amount of one or more toxicologically-acceptable gellants. In one embodiment the level of the gellant agent ranges from 0.01% to 40%, in another embodiment from 1% to 10%, in another embodiment from 2% to 5%, by weight of the article. Suitable gellant agents of the present invention include, but are not limited to, polyvinylpyrrolidone/eicosene copolymer sold under the tradename Ganex V-220F from ISP; tricontanyl polyvinylpyrrolidone sold under the tradename Ganex WP-660 from ISP; polyamide gallants including Sylvaclear, Sylvacote, Sylvagel, Uniclear all available from Arizona Chemical including Sylvaclear Lightwax; Sylvaclear PA 20; Sylvaclear PA 30; Sylvaclear PA 50; Sylvacote 2228; Sylvacote 2228E; Sylvagel 5000; Sylvagel 6000; Uniclear 100; Uniclear 100VG; Uniclear 80; Uniclear 80V; and mixtures thereof.

### Flavors, Fragrance, Sensates

The articles of the present invention may also include one or more components which provide flavor, fragrance, and/or sensate benefit (warming or cooling agents). Suitable components include menthol, wintergreen oil, peppermint oil, spearmint oil, leaf alcohol, clove bud oil, anethole, methyl salicylate, eucalyptol, cassia, 1-8 menthyl acetate, sage, eugenol, parsley oil, oxanone, alpha-irisone, marjoram, lemon, orange, propenyl guaethol, cinnamon, vanillin, thymol, linalool, cinnamaldehyde glycerol acetal knowm as CGA, and mixtures thereof, as well as coolants. The coolant can be any of a wide variety of materials. Included among such materials are carboxamides, menthol, ketals, diols, and mixtures thereof. In one embodiment the coolants in the present articles are selected from the group consisting of the paramenthan carboxyamide agents such as N-ethyl-p-menthan-3-carboxamide, known commercially as "WS-3", N,2,3-trimethyl-2-isopropylbutanamide, known as "WS-23," and mixtures thereof. Additional preferred coolants are selected from the group consisting of menthol, 3-1-menthoxypropane-1,2-diol known as TK-10 manufactured by Takasago, menthone glycerol acetal known as MGA manufactured by Haarmann and Reimer, and menthyl lactate known as Frescolat® manufactured by Haarmann and Reimer. The terms menthol and menthyl as used herein include dextro- and levorotatory isomers of these compounds and racemic mixtures thereof. TK-10 is described in U.S. Pat. No. 4,459,425, Amano et al., issued 7/10/84. WS-3 and other agents are described in U.S. Pat. No. 4,136,163, Watson, et al., issued Jan. 23, 1979. These agents may be present at a level of from 0% to 40%, in another embodiment from 0.05 to 5%, and in another embodiment from 0.1 to 2%, by weight of the article.

### Other Optional Ingredients.

The denture adhesive articles may also comprise one or more therapeutic actives suitable for topical administration. Therapeutic actives may be present at a level of from 0% to 70%, by weight of the article, and in one embodiment from 1% to 20% by weight of the article. Therapeutic actives include antimicrobial agents such as iodine, triclosan, peroxides, sulfonamides, bisbiguanides, or phenolics; antibiotics such as tetracycline, neomycin, kanamycin, metronidazole, cetylpyridium chloride, domiphen bromide, or clindamycin; anti-inflammatory agents such as aspirin, acetaminophen, naproxen and its salts, ibuprofen, ketorolac, flurbiprofen, indomethacin, eugenol, or hydrocortisone; dentinal desensitizing agents such as potassium nitrate, strontium chloride or sodium fluoride; fluorides such as sodium fluoride, stannous fluoride, MFP; anesthetic agents such as lidocaine or benzocaine; anti-fungals such as those for the treatment of *candida albicans;* aromatics such as camphor, eucalyptus oil, and aldehyde derivatives such as benzaldehyde; insulin; steroids; herbal and other plant derived remedies; and baking soda. It is recognized that in certain forms of therapy, combinations of these agents in the same delivery system may be useful in order to obtain an optimal effect. Thus, for example, an antimicrobial and an anti-inflammatory agent may be combined in a single delivery system to provide combined effectiveness.

Other Suitable ingredients include colorants, preservatives (such as methyl and propyl parabens), thickeners such as silicon dioxide, and polyethylene glycol. Colorants, preservatives, thickeners may be present at levels of from 0% to 20%, by weight of the article, in another embodiment from 0.1% to 10%, by weight.

Additionally, the articles may also comprise one or more solvents. These optional solvents may be miscible with the water insoluble component and/or be capable of being dissipated in-situ. In one embodiment these solvents may be dissipated in-situ by evaporation, dissolution, dispersion, bio-absorption, or any other suitable means. In another embodiment these solvents may be dissipated in-situ to leave behind a denture adhesive article. Such solvents may include materials with a viscosity ranging from 0.01, 0.1, 1, 5 centipoise 20°C, to 5, 10, 100, 1000 centipoise at 20°C in any combination of these levels. In one embodiment these solvents may be silicones, hydrocarbons, iso-dodecane, iso-hexadecane, iso-eicosane, and/or polyisobutene. Suitable grades of solvents include the Permethyl series (sold by Prespers Inc., New Jersey) such as Permethyl 97A, 99A, 101A, 102A, and mixtures thereof.

### PROCESS FOR PREPARATION OF THE ARTICLE

The articles utilized in accordance with the invention are formed by processes conventional in the arts, e.g. the film making industries such as casting, coating, calendaring, extrusion. In one embodiment the separate components of the article are melted and then blended in a mixing tank until a homogeneous mixture is achieved. Thereafter, the melted mixture may be cast to an acceptable thickness, on an appropriate substrate. Examples of such substrates include Mylar, continuous moving stainless steel belt (which may eventually entering a dryer section if needed), release paper and the like. The articles are then cooled. The articles may then be dried if needed, e.g. in a forced-air oven. The temperature of the drying air and length of drying time depend on the nature of the solvent utilized as is recognized in the art. Generally, the drying temperatures include a temperature between 25°C and 140°C, in another embodiment from 60° and 90° C for a duration of 20 minutes to 60 minutes, in another embodiment from 30 to 40 minutes. The article may then be cut into desired shapes with desired dimensions and then stacked and/or subsequently packaged.

In one embodiment, after processing, the article is then die-cut into desired shapes. These shapes may facilitate application of the article to the dentures.

In one embodiment in particular the present article is processed as follows: 1. melt the wax component; 2. mix the AVE/MA salt(s) with any other adhesive component; 3. add the adhesive mixture to the wax melt; 4. stir to made a homogeneous mixture; 5. pour the homogeneous mixture into mold or onto a suitable surface; 6. cool the mixture until it solidifies; 7. remove from substrate or mold or cut into the desired shape.

Another conventional film-making process known in the art is extrusion. This method is possible with films wherein the film forming ingredient comprises a variety of extrudable materials. The mechanical particulars of the extrusion process, e.g. the particular equipment utilized, the extruding force, the shape and temperature of the orifice and/or dies are considered to be within the skill of the art and can be varied in a known manner to achieve the physical characteristics of the articles described herein.

In one embodiment the thickness of the articles herein is generally between 0.1 mm to 2.5 mm, in another embodiment is from 0.4 mm to 1.5 mm thick, in another embodiment is from 0.5 mm to 1mm thick. The article may be thicker or thinner depending on the degree of cushioning desired by the user or wearer.

In one embodiment the articles herein may optionally be multiphase or have visually distinct phases. In another embodiment the articles herein may optionally have a release liner.

### ARTICLE USE

The present articles are generally applied to the denture prosthesis and thereafter the denture is secured to the oral cavity. In one embodiment the dentures are dried prior to application of the article. In one embodiment it is not necessary to wet the article and/or the denture prosthesis prior to applying it to the denture prosthesis in order to make the article stick to the denture prosthesis. The article may be applied to any suitable location on the prosthesis. In one embodiment the denture wearer generally wears the article from 1 hour to 3 days, in another embodiment from 6 hours to 24 hours. After usage the prosthesis is removed from the oral cavity, and any remaining article may be cleaned from the prosthesis, for example by gentle scrubbing with water and a brush. In one embodiment the articles can be combined into a kit, and the kit can further comprise an indicia which indicates to the user not to wet the product prior to application to the denture or oral surface (as shown in Fig. 16).

The following examples further describe and demonstrate embodiments within the scope of the present invention. The examples are given solely for the purpose of illustration and are not to be construed as limitations of the present invention. Many variations of these are possible without departing from the spirit and scope of the invention.

### EXAMPLES

### EXAMPLE I

| | A | B | C | D | E | F | H | I |
|---|---|---|---|---|---|---|---|---|
| | Grams | Grams | Grams | Grams | Grams | Grams | Grams | Grams |
| Ca/Zn AVE/MA Salt | 33.00 | 33.00 | 33.00 | 53.00 | | 10.00 | 28 | 24.5 |
| CMC | 20.00 | 20.00 | 20.00 | | 53.00 | 10.00 | 15 | 28.5 |
| AVE/MA Acid S-97 | | | 1.00 | | | | | |
| Microcrystalline Wax¹ W-835 | 46.92 | 47.00 | 47.00 | 47.00 | 47.00 | 80.00 | 45.52 | 46.92 |
| Flavors | | | 0.50 | | | | 0.4 | |
| Sacharrin | 00.08 | | 0.16 | | | | 0.08 | 0.08 |
| Colorants | | | 0.10 | | | | | |
| Silica | | | | | | | 1.00 | |
| Corn Starch | | | | | | | 10 | |

| | | | | | | | | |
|---|---|---|---|---|---|---|---|---|
| ¹Multiwax W 835 manufactured by Witco (Crompton, Sonneborn). The Multiwax W 835 may also be substituted with either Polyethylene A-C 1702 or Polyethylene A-C 6702. | | | | | | | | |

The Microcrystalline Wax W-835 (or Polyethylene A-C 1702 or Polyethylene A-C 6702) is melted, and the other ingredients are blended with it. The mixture is then made into sheets by any suitable means such as extrusion or rolling into sheets of suitable thickness such as 0.50mm, 0.67mm, 0.73mm, or 1.0mm. The sheet is then cut into shapes having a either a length of 45mm, and a width of 13 mm (at widest section) asymmetrical concave kidney shape or a length of 45mm, and a width of 7 mm concave symmetrical shape. These articles are suitable for application to dentures.

In the above example articles, all or part of the Ca/Zn AVE/MA salt may be substituted with Mg/Zn/Na AVE/MA Salts and/or Ca/Na AVE/MA salts; all or part of the CMC may be substituted with caraggeenan, and/or suitable cellulose derivatives; all or part of the Microcystalline Wax W-835 may be substituted with Polyethylene A-C 1702 (available from Honeywell), and/or Polyethylene A-C 6702 (available from Honeywell); and/or, the amount of each ingredient may also be increased or decreased by up to about 10%. Each of the above examples may be blended with each other prior to making into sheets; and/or used in conjunction with each other to form multilayer articles.

### EXAMPLE II

| | A | B |
|---|---|---|
| | Grams | Grams |
| Ca/Zn AVE/MA Salt | 33.00 | 33.00 |
| CMC | 20.00 | 20.00 |
| AVE/MA Acid S-97 | 1.00 | |
| Microcrystalline Wax² W-180 | 30.00 | 23.50 |
| Flavors | 0.50 | |
| Sacharrin | 0.16 | |
| Colorants | 0.10 | |
| Petrolatum | 17.00 | 23.50 |

| | | |
|---|---|---|
| ² Multiwax W 180 manufactured by Witco (Crompton, Sonneborn) | | |

The Microcrystalline Wax W-180 is melted, and the other ingredients are blended with it. The mixture is then made into sheets by any suitable means such as extrusion or rolling into sheets of suitable thickness such as 0.50mm, 0.67mm, 0.73mm, or 1.0mm. The sheet is then cut into shapes having a either a length of 45mm, and a width of 13 mm (at widest section) asymmetrical concave kidney shape or a length of 45mm, and a width of 7 mm concave symmetrical shape. These articles are suitable for application to dentures.

In the above example articles, all or part of the Ca/Zn AVE/MA salt may be substituted with Mg/Zn/Na AVE/MA salts and/or Ca/Na AVE/MA salts; all or part of the CMC may be substituted with HEC, Caraggeenan, and/or Karaya Gum; all or part of the Microcystalline Wax W-180 may be substituted with Microcrystalline Wax W-445 (also available from Witco), all or part of the Petrolatum may be substituted with Mineral Oil, and/or Polybutene; and/or the amount of each ingredient may also be increased or decreased by up to about 10%. Each of the above examples may be blended with each other prior to making into sheets; and/or used in conjunction with each other to form multilayer articles.

### EXAMPLE III

| | A | B | C |
|---|---|---|---|
| | Grams | Grams | Grams |
| Ca/Zn AVE/MA Salt | 33 | 33 | 33 |
| CMC | 20.00 | 20.00 | 20 |
| Saccharin | 0.04 | 0.06 | 0.02 |
| Silica | 0.57 | 0.29 | 0.86 |
| Mineral Oil | 11.98 | 5.99 | 17.96 |
| Microcrystalline Wax³ W-835 | 23.46 | 35.19 | 11.73 |
| Petrolatum | 10.96 | 5.48 | 16.43 |

| | | | |
|---|---|---|---|
| ³ Crompton, Sonneborn Multiwax W 835 manufactured by Witco (Crompton, Sonneborn). The Multiwax W 835 may also be substituted with either Polyethylene A-C 1702 or Polyethylene A-C 6702. | | | |

The Microcrystalline Wax W-835 is melted, and the other ingredients are blended with it. A unit dose of the mixture is then extruded into an array of foil pouches for packaging into a denture adhesive product. The denture wearer may then apply the unit dose mixture onto the denture and mold it into the desired shape or shapes.

In the above example, all or part of the Ca/Zn AVE/MA salt may be substituted with Mg/Zn/Na AVE/MA salts and/or Ca/Na AVE/MA salts; all or part of the CMC may be substituted with HEC, Caraggeenan, and/or Karaya Gum; all or part of the Microcystalline Wax W-835 may be substituted with Polyethylene A-C 1702 (available from Honeywell), and/or Polyethylene A-C 6702 (available from Honeywell); all or part of the Petrolatum may be substituted with Mineral Oil, and/or Polybutene.

### Example IV

| | A | B | C | D* |
|---|---|---|---|---|
| | % | % | % | % |
| Ca/Zn AVE/MA Salt | 33 | 33 | 33 | 33 |
| CMC | 20 | 20 | 20 | 20 |
| Silica | 1.03 | 0.97 | 0.86 | 1.14 |
| Mineral Oil | 21.56 | 20.36 | 17.96 | 23.95 |
| Petrolatum | 19.72 | 18.62 | 16.43 | 21.91 |
| Sacharin | 0.01 | 0.01 | 0.02 | 0.00 |
| Microcrystalline Wax³ W-835 | 4.69 | 7.04 | 11.73 | 0.00 |

| | | | | |
|---|---|---|---|---|
| * Reference formula IV-D is for comparative purposes only ³ Crompton, Sonneborn Multiwax W 835 manufactured by Witco (Crompton, Sonneborn). The Multiwax W 835 may also be substituted with either Polyethylene A-C 1702 or Polyethylene A-C 6702. | | | | |

To make the above prototypes IV-A-D, the wax is melted at about 95C (in examples A-C) and the other ingredients are blended with it in a heated vessel at about 65C under vacuum. The blended products can be filled into tubes. The product can be squeezed out of the tubes onto a denture in the form of ribbons. The ribbons from products IV-A-C can be picked up, and say placed at the desired location, with little or no residue on the fingers. The ribbons from product IV-D cannot be picked up and placed since they are adhered to the denture surface and leave substantial residue on the fingers.

### EXAMPLE-V

| **Sample** | **PEG 600** | **PEG 1000** | **CMC 7HF** | **Ca/Zn Gantrez** | **Caraqeenan** | **Xantha n** |
|---|---|---|---|---|---|---|
| A | 11.75 | 35.25 | | | | 53 |
| B | 11.75 | 35.25 | | | 53 | |
| C | 11.75 | 35.25 | 20 | 33 | | |
| D | 9.4 | 37.6 | 20 | 33 | | |
| E | 35.25 | 11.75 | 20 | 33 | | |
| F | 4.7 | 42.3 | 20 | 33 | | |

The above formulas can be made by the following procedure: PEG 1000 is heated at approximately 50 C on a hot plate to melt it. The molten PEG 1000 is then mixed with the appropriate amount of PEG 600 (a liquid at room temperature). While still warm, the molten solutions are mixed with CMC, Gantrez, Carregeenan, and/or Xanthan gum depending on the specific formulation. After mixing, the formulations are pressed at room temperature under 9000 lb of pressure for approximately 2 minutes. The samples are then removed from the press and can be cut into concaved shapes.

### EXAMPLE VI

| **Sample** | **CMC 7LF** | **Glycerin** | **DI H2O** |
|---|---|---|---|
| A | 0.1 | 0.05 | 9.85 |
| B | 0.1 | 0.1 | 9.8 |
| C | 0.1 | 0 | 9.9 |
| D | 0.1 | 0.25 | 9.65 |

The above formulas can be made by the following procedure: CMC is mixed with water and glycerin on a stir plate with high magnetic stirring to create a vortex. After approximately 20 minutes of stirring to allow the CMC to dissolve, the samples are cast onto Teflon boats. The samples are then placed in an oven under vacuum at approximately 50 C overnight to remove water. The samples are then removed from the Teflon boats and can be cut into concaved shapes.

### TEST METHODS

The bioerosion of the inventive articles can be measured by the following method: run a water source on top of the sample specimen for about 30 minutes while the specimen sits atop a wire mesh. The water source is a laboratory faucet adjusted such that the temperature is 39 ± 1 °C and the flow rate is 16 ± 1 ml/sec. Use a funnel to focus the flow and help dampen the effect of small pressure and temperature fluctuation within the water lines. The wire mesh grid has square openings approximately (0.2286cm x 0.2286cm) and is placed (6.35 cm) below the tip of the funnel where it is clamped to a metal ring for support. Sample specimens weighing 0.025 g are placed on the mesh and images are taken at 0, 10 and 30 minutes to follow bio-erosion of the specimen. After 30 minutes the wire mesh containing the remainder of the specimen is removed and heated for 1 hour at 60 °C under vacuum to remove all remaining water. After the heating period, final weights are taken to calculate weight loss due to bio-erosion. An average of 3 specimens per sample are used to calculate bio-erosion time and weight loss. The article is bioerodible if it does not leave behind visible residue, film, or sheet after about 30 minutes under these testing conditions., and/or if it cannot be easily separated or peeled away manually in one or more large pieces after 30 minutes under these testing conditions, and/or if it leaves behind less than 2, less than 4, less than 6, and/or less than 8% by wight of residue (of the original weight of the article) after 30 minutes under these testing conditions. The above bio-erosion test may also be conducted at various time-points up to 8 hours.

The dry tack can be measured by the following method: 1. remove the article from the package material; 2. place the article on the palate-portion of a dry, acrylic upper-denture with the teeth facing downward; 3. apply pressure with fingers for 3 to 10 seconds; 4. thereafter remove finger pressure; 5. then invert the denture with the teeth facing upward. In one embodiment the article demonstrates dry tack if: i. The article does not stick to fingers during steps 1-2, ii. leaves little or no residue on the fingers in steps 3-4, and iii. in step-5, the article does not fall off of the denture, once inverted, for at least 10-30 seconds, or at least about 1 minute.

In another embodiment the article demonstrates dry tack if: i. The article does not stick to fingers during steps 1-4, and ii. in step-5, the article does not fall off of the denture, once inverted, for at least 10-30 seconds, or at least 1 minute.

In another embodiment the article demonstrates dry tack if in step-5, the article does not fall off of the denture, once inverted, for at least about 10-30 seconds, or at least about 1 minute.

The dry tack of the inventive articles can also be measured by the following procedure:
a. Compress a 5mm diameter disc (0.67mm thick) sample of the article between a 1" diameter cylindrical probe (made from polymethylmethacrylate) and a flat sheet of polymethylmethacrylate with a 2000 gram-force for 2 seconds,
b. Pull off the probe at 1mm/second and record peak force,
c. Repeat procedure with no sample sandwiched between the two surfaces, and
d. Calculate: Dry Tack in grams/square centimeter = (Peak Force with Sample - Peak Force without sample) / Cross sectional area of sample disc.
In one embodiment the above procedure is repeated with an applied force of 250 gram-force in step-a and the tack measured in steps b-d;
The article has dry tack if the tack measured with a 250 gram-force applied force is less than about 25, 50, 100, 200, or 500 grams/square-centimeter, and the tack measured with a 2000 gram-force applied force is greater than about 200, 500, 1000, 2000, 5000, 10000, or 25000 grams/square-centimeter, and any combination of these levels.
The modulus G' of the inventive article can be measured by the following procedure:
a. Load a sample disc of 8mm diameter and 0.67mm thickness onto a ARES rheometer using a parallel plate fixture with a compressive force of 500 grams. If the sample is flowable, a sufficient amount of material is used to fill the 1 mm gap on a 25 mm diameter parallel plate fixture.
b. Set strain to be 0.02%, c. Measure G' at a sweep of frequencies including .1 Hz.

The normalized dislodgement force and dislodgement force ratio of the inventive article can be measured by the following method:
Instrument: An Instron model 5544 is used. The load cell is calibrated according to manufacturer's specifications annually. The choice of load cell is determined by having the forces generated by the adhesive fall within the recommended operating range for the load cell. This is typically between 10% - 90% of full capacity.
Test Fixtures: The geometry of a cylindrical probe and a flat plate are used as the test fixtures. The probe is made from PMMA, 0.2 sq.cm to 10 sq.cm in surface area. For the base plate, the same PMMA material is used but in sheet form, ¼" thick. This is cut into 6" x 6" plates to be clamped onto the Instron.
Hydrating Liquid: Artificial saliva containing low levels of various salts is used to hydrate the adhesive.

### Artificial Saliva Composition

| Ingredient | | Amount per Liter |
|---|---|---|
| K₂HPO₄ | | 4.2 g |
| KH₂PO₄ | | 3.2 g |
| KOH | | 2 pellets |
| Mineral Stock Solution | | 5 ml |
| - KCI | 8 g per 100 ml of Stock | |
| Solution | | |
| -NaCl | 8 g | |
| - Na2SO4 | 0.264 g | |
| - MgCl2.6H2O | 0.7687 | |
| (or 0.36 g Anhydrous MgCI2) | | |

Adhesive: 0.1 to 1.0 gram of adhesive is applied to the probe.
Hydration: The hydrating liquid (0.2 mL of artificial saliva to 2.0 ml) is pipetted onto the surface of the adhesive. The assembly is then permitted to hydrate for 20 minutes or more.
Test Method: Once the sample is hydrated, it is mounted onto the Instron and the test is carried out via computer control. The method is comprised of the following steps:
(a) Compression to 750 to 7500 g of force
(b) Hold at compression for 2 minutes
(c) Reduce compressive force to 200 g_{f}
(d) Hold (1 minute)
(e) Pull off at 1 mm/s
(f) Record Peak Dislodgement Force
(g) Calculate "Normalized Dislodgement Force" = (Peak Dislodgement Force) / (Surface Area of Probe); report in grams force per sq.cm
(h) Repeat steps A-F for commercial Fixodent Original denture adhesive (available commercially manufactured by P&G), or for the following reference formula: Ca(47.5%)/Zn(17.5%) MVE/MA salt 33%, sodium carboxymethylcellulose 20%, mineral oil USP (65-75cst at 40C) 23.93%, petrolatum USP (consistency 17-20mm) 21.87%, colloidal silicon dioxide 1.14%, and Opatint OD1646 0.06%; suitable methods to make this reference formula are disclosed in US 5,073,604, Holeva K., and US 6,617, 374 Rajaiah J.
   (i) Calculate "Dislodgement Force Ratio" = (Peak Dislodgement Force of Prototype Adhesive) / (Peak Dislodgement Force of Fixodent Original) '
      Data: Each sample is repeated a minimum of 3 times and the average value of the "Normalized Dislodgement Force" and "Dislodgement Force Ratio" are reported.

Specifically the normalized dislodgement force and dislodgement force ratio can be measured by using the following parameters in the procedure: 0.25 gram adhesive; 1 inch diameter probe; hydration time of 20 minutes; and compression force of 7500 grams.

The "normalized ooze amount" and "ooze ratio" of the inventive article can be measured by the following procedure:
a. Load initial sample weight of about 0.50 grams uniformly onto a 1 inch diameter cylindrical probe made from polymethylmethacrylate,
b. Bring probe to 1.2 mm of base plate, also made from polymethylmethacrylate, c. Apply 750 gram force for 90 seconds,
d. At 90 seconds, trim and weigh material that has oozed out,
e. Calculate "Normalized Ooze Amount" = (Amount oozed out / Initial sample weight) x 100,
f. Repeat Steps a-e using commercial Fixodent Original a denture adhesive cream commercially manufactured by P&G, or with the following reference formula: Ca(47.5%)/Zn(17.5%) MVE/MA salt 33%, sodium carboxymethylcellulose 20%, mineral oil USP (65-75cst at 40C) 23.93%, petrolatum USP (consistency 17-20mm) 21.87%, colloidal silicon dioxide 1.14%, and Opatint OD1646 0.06%; suitable methods to make this reference formula are disclosed in US 5,073,604, Holeva K., and US 6,617, 374 Rajaiah J.,
g. Calculate "Ooze Ratio" = Normalized Ooze Amount of Prototype Adhesive /Normalized Ooze Amount of Fixodent Original,
h. Each sample is repeated a minimum of 3 times and the average value of the "Normalized Ooze Amount" and "Ooze Ratio" are reported.

## Claims

1. The use of at least two denture adhesive articles for application to the grooves of a denture, wherein each article is a concave shaped sheet and comprises a homogeneous mixture of:
a) a water insoluble thermoplastic component selected from rubber, elastomers, plastomers, natural wax, synthetic wax, polyvinyl chloride, nylon, fluorocarbon, polyurethane prepolymer, polyethylene, polystyrene, polypropylene, cellulosic resins, acrylic resins, petrolatum, and mixtures thereof; and
b) a denture adhesive component.

2. The use of Claim 1 wherein the articles are kidney shaped.

3. The use of Claim 1 wherein the ratio of width to length of each article is from 1:1 to 1:10, preferably from 1:5 to 1:3.

4. The use of Claim 1 wherein the thickness of each article is from 0.1 mm to 2.5 mm, preferably from 0.4 mm to 1.5 mm.

5. The use of Claim 1 wherein the articles have minimal or no adhesive properties in the dry state until activated by pressure applied by a user.

6. The use of Claim 1 wherein the articles are flexible.

7. The use of Claim 1 wherein the denture adhesive component is a salt or mixed salt of AVE/MA; preferably wherein the denture adhesive component is selected from a salt or mixed salt of AVE/MA, the salt containing a cationic salt function comprising a cation selected from Group IA and Group 2A cations of the periodic table, yttrium, titanium, zirconium, vanadium, chromium, manganese, iron, nickel, copper, zinc, boron, aluminum, and mixtures thereof; more preferably wherein the salt is selected from a calcium/zinc salt of AVE/MA, a magnesium/zinc/sodium salt of AVE/MA, a calcium/sodium salt of AVE/MA, a zinc salt of AVE/MA and mixtures thereof.

8. The use of Claim 1 wherein the articles are bioerodible.

9. The use of Claim 1, wherein the water insoluble thermoplastic component is selected from the group consisting of polyethylene, microcrystalline wax, or mixtures thereof.

## Patentansprüche

1. Verwendung von mindestens zwei Zahnprothesen-Haftmittelartikeln zur Applikation auf die Rillen einer Zahnprothese, wobei jeder Artikel eine konkav geformte Schicht ist und eine homogene Mischung aus Folgendem umfasst:
a) einem wasserunlöslichen thermoplastischen Bestandteil, der ausgewählt ist aus Kautschuk, Elastomeren, Plastomeren, natürlichem Wachs, synthetischem Wachs, Polyvinylchlorid, Nylon, Fluorkohlenstoff, Polyurethanpräpolymer, Polyethylen, Polystyrol, Polypropylen, Celluloseharzen, Acrylharzen, Petrolatum und Mischungen davon, und
b) einem Zahnprothesen-Haftmittelbestandteil.

2. Verwendung nach Anspruch 1, wobei die Artikel nierenförmig sind.

3. Verwendung nach Anspruch 1, wobei das Verhältnis von Breite zu Länge jedes Artikels 1:1 1 bis 1:10, vorzugsweise 1:5 bis 1:3 beträgt.

4. Verwendung nach Anspruch 1, wobei die Dicke jedes Artikels 0,1 mm bis 2,5 mm, vorzugsweise 0,4 mm bis 1,5 mm beträgt.

5. Verwendung nach Anspruch 1, wobei die Artikel in trockenem Zustand minimale oder gar keine Hafteigenschaften aufweisen, bis sie durch Druck, der von einem Benutzer ausgeübt wird, aktiviert werden.

6. Verwendung nach Anspruch 1, wobei die Artikel flexibel sind.

7. Verwendung nach Anspruch 1, wobei der Zahnprothese-Haftmittelbestandteil ein Salz oder ein gemischtes AVE/MA-Salz ist, wobei der Zahnprothese-Haftmittelbestandteil ausgewählt ist aus einem AVE/MA-Salz, wobei das Salz eine kationische Salzfunktion enthält, die ein Kation umfasst, das aus Kationen von Gruppe IA und Gruppe 2A der Tabelle des Periodensystems ausgewählt ist, Yttrium, Titan, Zirkonium, Vanadium, Chrom, Mangan, Eisen, Nickel, Kupfer, Zinke, Bor, Aluminium und Mischungen davon, wobei das Salz stärker bevorzugt ausgewählt ist aus einem Calcium/Zink-Salz von AVE/MA, einem Magnesium/Zink/Natrium-Salz von AVE/MA, einem Calcium/Natrium-Salz von AVE/MA, einem Zinksalz von AVE/MA und Mischungen davon.

8. Verwendung nach Anspruch 1, wobei der Artikel biologisch erodierbar ist.

9. Verwendung nach Anspruch 1, wobei der wasserunlösliche thermoplastische Bestandteil ausgewählt ist aus der Gruppe, bestehend aus Polyethylen, mikrokristallinem Wachs oder Mischungen davon.

## Revendications

1. Utilisation d'au moins deux articles adhésifs pour dentier pour application sur les rainures d'un dentier, dans laquelle chaque article est une feuille de forme concave et comprend un mélange homogène de :
a) un composant thermoplastique insoluble dans l'eau choisi parmi le caoutchouc, des élastomères, des plastomères, de la cire naturelle, de la cire synthétique, du chlorure de polyvinyle, du nylon, un fluorocarbure, un prépolymère de polyuréthane, du polyéthylène, du polystyrène, du polypropylène, des résines cellulosiques, des résines acryliques, du pétrolatum, et leurs mélanges ; et
b) un composant adhésif pour dentier.

2. Utilisation selon la revendication 1, dans laquelle les articles sont en forme de haricot.

3. Utilisation selon la revendication 1, dans laquelle le rapport de la largeur sur la longueur de chaque article va de 1:1 à 1:10, de préférence de 1:5 à 1:3.

4. Utilisation selon la revendication 1, dans laquelle l'épaisseur de chaque article va de 0,1 mm à 2,5 mm, de préférence de 0,4 mm à 1,5 mm.

5. Utilisation selon la revendication 1, dans laquelle les articles ont des propriétés adhésives minimales ou nulles à l'état sec jusqu'à ce qu'ils soient activés par une pression appliquée par un utilisateur.

6. Utilisation selon la revendication 1, dans laquelle les articles sont souples.

7. Utilisation selon la revendication 1, dans laquelle le composant adhésif pour dentier est un sel ou un sel mixte d'AVE/MA (copolymère alkyl-éther vinyle/acide ou anhydride maléique) ; de préférence dans laquelle le composant adhésif pour dentier est choisi parmi un sel ou un sel mixte d'AVE/MA, le sel contenant une fonction sel cationique comprenant un cation choisi parmi les cations du Groupe IA et du Groupe 2A du tableau périodique, yttrium, titane, zirconium, vanadium, chrome, manganèse, fer, nickel, cuivre, zinc, bore, aluminium, et leurs mélanges ; plus préférablement dans laquelle le sel est choisi parmi un sel de calcium/zinc d'AVE/MA, un sel de magnésium/zinc/sodium d'AVE/MA, un sel de calcium/sodium d'AVE/MA, un sel de zinc d'AVE/MA et leurs mélanges.

8. Utilisation selon la revendication 1, dans laquelle l'article est susceptible de bio-érosion.

9. Utilisation selon la revendication 1, dans laquelle le composant thermoplastique insoluble dans l'eau est choisi dans le groupe constitué de polyéthylène, cire microcristalline, ou leurs mélanges.
